(19) Europäisches Patentamt
European Patent Office
Office européen des brevets

(11) Veröffentlichungsnummer: **0 242 767 B1**

# (12) EUROPÄISCHE PATENTSCHRIFT

(45) Veröffentlichungstag der Patentschrift: **11.09.91**

(51) Int. Cl.$^5$: **C07D 209/16**

(21) Anmeldenummer: **87105464.9**

(22) Anmeldetag: **13.04.87**

## (54) N-dihydroindolylethylsulfonamide.

(30) Priorität: **23.04.86 DE 3613623**

(43) Veröffentlichungstag der Anmeldung:
**28.10.87 Patentblatt 87/44**

(45) Bekanntmachung des Hinweises auf die Patenterteilung:
**11.09.91 Patentblatt 91/37**

(84) Benannte Vertragsstaaten:
**AT BE CH DE ES FR GB IT LI NL SE**

(56) Entgegenhaltungen:
**EP-A- 0 201 735**

**JOURNAL OF PHARMACEUTICAL SCIENCES, Band 60, Nr. 4, April 1971, Seiten 636-637; B.T. HO et al.: "Hydroxyindole-O-methyltransferase VI: Inhibitory activities of substituted Benzoyltryptamines and Benzenesulfonyltryptamines"**

**JOURNAL OF MEDICINAL CHEMISTRY, Band 14, Nr. 6, 1971, Seiten 553-554; B.T. HO et al.: "Central nervous system depressive activity of some amides of Tryptamine 1"**

(73) Patentinhaber: **BAYER AG**

**W-5090 Leverkusen 1 Bayerwerk(DE)**

(72) Erfinder: **Böshagen, Horst, Dr.**
**Wiesenstrasse 4**
**W-5657 Haan(DE)**
Erfinder: **Rosentreter, Ulrich, Dr.**
**Kondorweg 23**
**W-5600 Wuppertal 1(DE)**
Erfinder: **Lieb, Volker, Dr.**
**Alfred-Kubin-Strasse 1**
**W-5090 Leverkusen 1(DE)**
Erfinder: **Oediger, Hermann, Dr.**
**Roggendorfstrasse 51**
**W-5000 Köln 80(DE)**
Erfinder: **Fiedler, Volker-Bernd, Dr.**
**Lehner Mühle 46**
**W-5090 Leverkusen 3(DE)**
Erfinder: **Perzborn, Elisabeth, Dr.**
**Am Tescher Busch 13**
**W-5600 Wuppertal 1(DE)**
Erfinder: **Seuter, Friedel, Dr.**
**Moospfad 16**
**W-5600 Wuppertal 1(DE)**

**EP 0 242 767 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue N-Dihydroindolylethyl-sulfonamide, Verfahren zu ihrer Herstellung und ihre Verwendung in Arzneimitteln.

Es wurden neue N-Dihydroindolylethyl-sulfonamide der allgemeinen Formel (I)

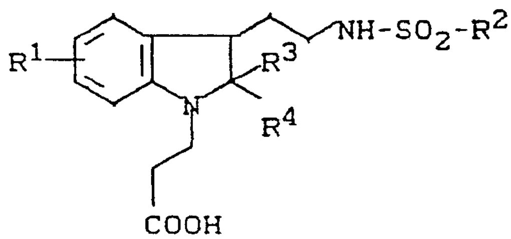

(I)

in welcher

$R^1$ - für Wasserstoff, Fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino, Acetylamino steht, oder
- für eine Gruppe der Formel $-OR^8$ steht,
worin
$R^8$- Wasserstoff, $C_1$-$C_4$-Alkyl, Phenyl oder Benzyl bedeutet,

oder

$R^1$ - für $C_1$-$C_4$-Alkyl steht,
$R^2$ - für Phenyl steht, das bis zu 2-fach gleich oder verschieden durch Fluor, Chlor, Brom, Cyano, Trifluormethyl, Trifluormethoxy, $C_1$-$C_4$-Alkoxy, Methylthio, Hydroxy, Methoxycarbonyl, Ethoxycarbonyl, Dimethylamino, Acetylamino, Diethylamino substituiert ist,
$R^3$ - für Wasserstoff oder $C_1$-$C_4$-Alkyl,
und
$R^4$ - für Wasserstoff steht,
oder
$R^3$ und $R^4$ gemeinsam einen Carbonylsauerstoff binden,
und deren Salze gefunden.

Die erfindungsgemäßen Stoffe haben mehrere asymmetrische Kohlenstoffatome und können daher in verschiedenen stereochemischen Formen existieren. Sowohl die einzelnen Isomeren als auch deren Mischungen sind Gegenstand der Erfindung.

Die erfindungsgemäßen N-Dihydroindolylethyl-sulfonamide können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze der N-Dihydroindolylethyl-sulfonamide können Metall- oder Ammoniumsalze der erfindungsgemäßen Stoffe welche eine freie Carboxylgruppe besitzen seine. Besonders bevorzugt sind z.B. Natrium-, Kalium-, Magnesium- oder Calciumsalze sowie Ammoniumsalze, die abgeleitet sind von Ammoniak oder organische Amine wie beispielsweise Ethylamin, Di-bzw. Triethylamin, Di- bzw. Triethanolamin, Dicyclohexylamin, Dimethylaminoethanol, Arginin oder Ethylendiamin.
Die erfindungsgemäßen Stoffe zeigen überraschenderweise eine thrombozytenaggregationshemmende Wirkung und können zur therapeutischen Behandlung von Menschen und Tieren verwendet werden.

Bevorzugt werden Verbindungen der allgemeinen Formel (I),
worin

$R^1$ - für Wasserstoff, Fluor, Chlor oder Brom steht,
$R^2$ - für Phenyl steht, das durch Flour, Chlor oder Brom substituiert ist,
$R^3$ - für Wasserstoff oder $C_1$-$C_4$-Alkyl,
und
$R^4$ - für Wasserstoff steht,
oder
$R^3$ und $R^4$ gemeinsam einen Carbonylsauerstoff binden,
und deren Salze.

Beispielhaft seien folgende N-Dihydroindolylethyl-sulfonamide genannt:

N-[2-[1(2-Carboxyethyl)-2,3-dihydro-1H-indol-3-yl]-ethyl]benzolsulfonamid
N[2-[1-(2-Carboxyethyl)-2,3-dihydro-1H-indol-3-yl]-ethyl]-(4-fluorphenyl)sulfonamid
N-[2-[1-(2-Carboxyethyl)-2,3-dihydro-2-methyl-1H-indol-3-yl]ethyl]-(4-chlorphenyl)sulfonamid
N-[2-[1-(2-Carboxyethyl)-2,3-dihydro-2-methyl-1H-indol-3-yl]ethyl]-(4-fluorphenyl)sulfonamid
N-[2-[1-(2-Carboxyethyl)-2,3-dihydro-5-fluor-2-methyl-1H indol-3-yl]ethyl]-(4-fluorphenyl)sulfonamid
N-[2-[1-(2-Carboxyethyl)-2,3-dihydro-2-oxo-1H-indol-3-yl]ethyl]benzolsulfonamid
N-[2-[1-(2-Carboxyethyl)-2,3-dihydro-2-oxo-1H-indol-3-yl]ethyl]-(4-chlorphenyl)sulfonamid
N-[2-[1-(2-Carboxyethyl)-2,3-dihydro-2-oxo-1H-indol-3-yl]ethyl]-(4-fluorphenyl)sulfonamid

Im Rahmen der vorliegenden Erfindung entsprechen die N-Dihydroindolylethyl-sulfonamide (Ia) der Formel

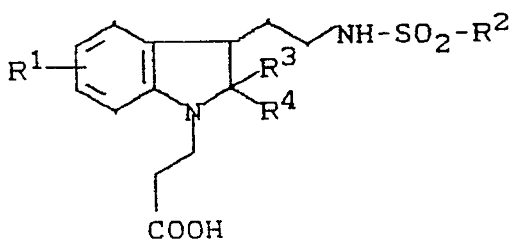

(Ia),

in welcher
$R^1$ und $R^2$ die angegebene Bedeutung haben,
$R^3$ - für Wasserstoff oder Alkyl steht,
und
$R^4$ - für Wasserstoff steht.

Im Rahmen der vorliegenden Erfindung entsprechen die N-Dihydroindolylethyl-sulfonamide (Ib) der Formel

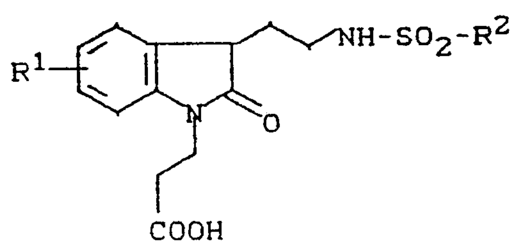

(Ib),

in welcher
$R^1$ und $R^2$ die angegebene Bedeutung haben.

Es wurde ein Verfahren zur Herstellung von N-Dihydroindolylethyl-sulfonamide der allgemeinen Formel (Ia)

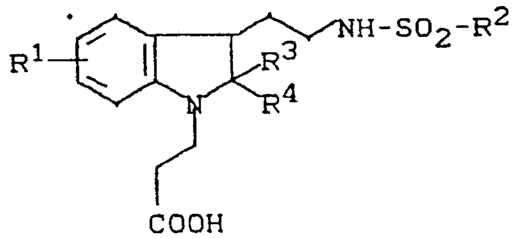

(Ia),

in welcher
$R^1$ und $R^2$ die angegebene Bedeutung haben,
$R^3$ - für Wasserstoff oder Alkyl steht
und

$R^4$ - für Wasserstoff steht

und deren Salze gefunden, das dadurch gekennzeichnet ist, daß man N-Indolylethyl-sulfonamide der allgemeinen Formel (II)

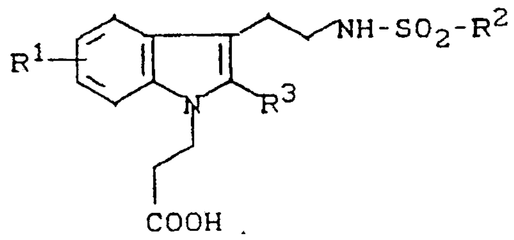

(II)

in welcher

$R^1$, $R^2$ und $R^3$ die angegebene Bedeutung haben,

oder deren Salze, gegebenenfalls in Gegenwart eines inerten organischen Lösungsmittels in Anwesenheit einer Säure und eines Reduktionsmittels hydriert,

gegebenenfalls Isomeren in üblicher Weise trennt und im Fall der Herstellung der Salze mit einer entsprechenden Base umsetzt.

Das erfindungsgemäße Verfahren kann durch das folgende Formelschema erläutert werden:

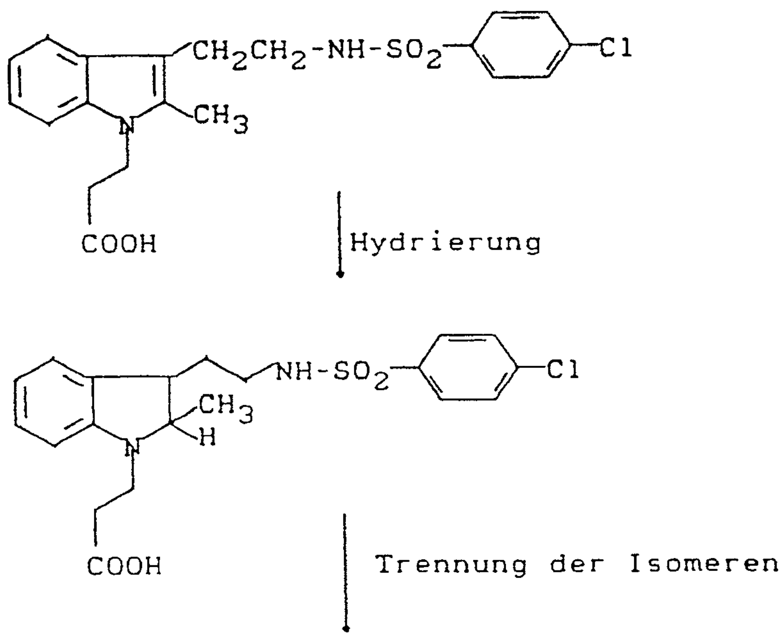

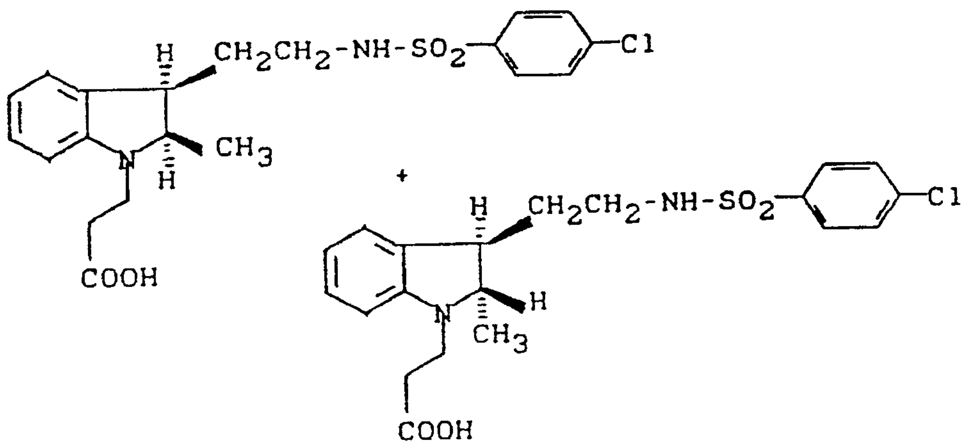

+

Als Lösungsmittel für die Hydrierung kommen inerte organische Lösungsmittel in Frage, die sich unter den Reaktionsbedingungen nicht verändern. Hierzu gehören bevorzugt Ether, wie Diethylether, Dioxan oder Tetrahydrofuran, oder Essigsäure, Trifluoressigsäure, Methansulfonsäure oder Trifluormethansulfonsäure. Ebenso ist es möglich Gemische der genannten Lösungsmittel einzusetzen.

Als Säuren können die üblichen organischen Säuren eingesetzt werden. Hierzu gehören vorzugsweise Carbonsäuren wie beispielsweise Essigsäure, Propionsäure, Chloressigsäure, Dichloressigsäure oder Trifluoressigsäure oder Sulfonsäuren, wie beispielsweise Methansulfonsäure, Ethansulfonsäure, Toluolsulfonsäure, Benzolsulfonsäure oder Trifluormethansulfonsäure.

Als Reduktionsmittel für die erfindungsgemäße Hydrierung kommen die üblichen Reduktionsmittel in Frage. Hierzu gehören vorzugsweise Hydride, wie z.B. Natriumborhydrid, Natriumcyanoborhydrid, Tetrabutylammoniumborhydrid, Tetrabutylammoniumcyanoborhydrid,Tributylzinnhydrid, Triethylsilan, Dimethylphenylsilan oder Triphenylsilan.

Die Hydrierung erfolgt im allgemeinen in einem Temperaturbereich von -40°C bis + 80°C, bevorzugt von - 20°C bis +60°C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Druckbereich von 0,5 bis 5 bar).

Im allgemeinen setzt man 1 bis 5, bevorzugt 1 bis 1,5 Mol des Reduktionsmittels, bezogen auf 1 Mol des N-Indolylethyl-sulfonamids (II) ein. Besonders bevorzugt setzt man molare Mengen der Reaktanden ein.

Bei der Durchführung des erfindungsgemäßen Verfahrens fallen die Verbindungen im allgemeinen als Isomerengemisch an, das nach üblichen Methoden, wie z.B, Kristallisation oder bevorzugt Chromatographie, in die einzelnen Isomeren getrennt werden kann [E.L. Eliel, "Stereochemistry of Carbon Compounds", McGraw Hill (1962); W.C. Still, M. Kahn, A. Mitra, J. Org. Chem. 43, 2923 (1978)].

Das erfindungsgemäße Verfahren kann beispielsweise wie folgt durchgeführt werden:
Das N-Indolylethylsulfonamid wird in einem inerten Lösungsmittel gelöst und anschließend das Reduktionsmittel portionsweise zugegeben. Nach Beendigung der Reaktion wird durch Extraktion und/oder Chromatographie aufgearbeitet.

Es wurde auch ein Verfahren zur Herstellung von N-Dihydroindolylethyl-sulfonamideder allgemeinen Formel (Ib)

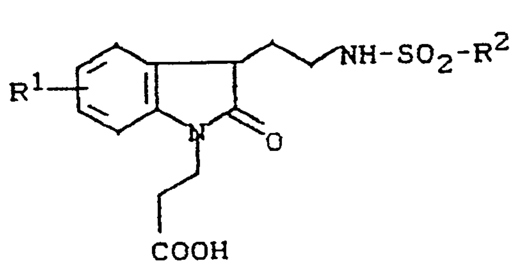

(Ib),

in welcher

$R^1$ und $R^2$ die angegebene Bedeutung haben,

und deren Salze, gefunden, das dadurch gekennzeichnet ist, daß man N-Indolylethyl-sulfonamide der allgemeinen Formel (IIa)

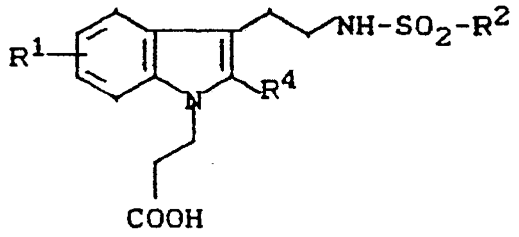

(IIa)

in welcher

$R^1$ und $R^2$ die oben angegebene Bedeutung haben, und

$R^4$ für Wasserstoff steht,

oder deren Salze,

in inerten Lösungsmitteln, gegebenenfalls in Anwesenheit einer Säure oxidiert und im Fall der Herstellung der Salze mit einer entsprechenden Base umsetzt.

Das erfindungsgemäße Verfahren kann durch das folgende Formelschema erläutert werden:

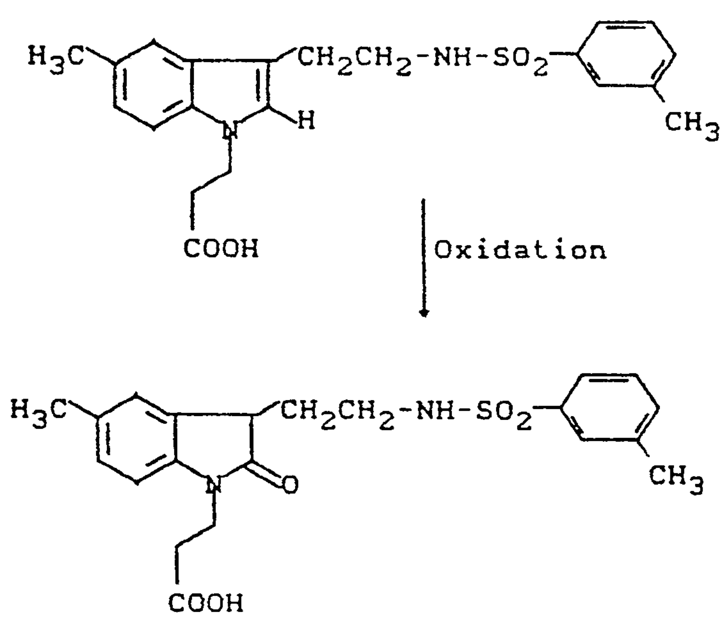


Oxidationsmittel für das erfindungsgemäße Verfahren sind bevorzugt elektrophile Oxidationsmittel. Als bevorzugte Oxidationsmittel seien N-Bromsuccinimid, Halogene, wie Chlor und Brom, und Dimethylsulfoxid genannt. Insbesondere bevorzugt werden Brom und Dimethylsulfoxid.

Besonders bevorzugt ist die Oxidation mit Dimethylsulfoxid in inerten Lösungsmitteln in Anwesenheit von Säuren.

Als Lösungsmittel kommen hierbei wasser oder die üblichen inerten organischen Lösungsmittel in Frage, die sich unter den Reaktionsbedingungen nicht verändern. Hierzu gehören bevorzugt: Alkohole wie Methanol, Ethanol, Propanol oder Isopropanol, oder Ether wie Diethylether, Dioxan, Tetrahydrofuran, Glykolmono- oder Glykoldimethylether, oder Kohlenwasserstoffe wie beispielsweise Benzol, Toluol, Xylol oder Erdölfraktionen (etwa $C_5$ bis $C_{12}$), oder Halogenkohlenwasserstoffe, wie beispielsweise Methylenchlorid, Chloroform, Tetrachlorkohlenstoff oder 1,2-Dichlorethan oder Essigsäure, Trifluoressigsäure oder Dimethylsulfoxid.

Die Oxidation kann gegebenenfalls in Gegenwart von Säuren durchgeführt werden.

Als Säuren können die üblichen anorganischen oder organischen Säuren eingesetzt werden. Hierzu gehören vorzugsweise anorganische Säuren wie Salzsäure, Bromwasserstoffsäure, Iodwasserstoffsäure, Schwefelsäure, Phosphorsäure oder organische Säuren wie Essigsäure, Trifluoressigsäure, Chloressigsäure, Dichloressigsäure, Trichloressigsäure oder Sulfonsäuren wie Methansulfonsäure, Ethansulfonsäure, Benzolsulfonsäure, Toluolsulfonsäure oder Trifluormethansulfonsäure.

Die Oxidation erfolgt im allgemeinen in einem Temperaturbereich von 0 °C bis 100 °C, bevorzugt von 20 °C bis 60 °C.

Das erfindungsgemäße Verfahren wird im allgemeinen bei Normaldruck durchgeführt, es ist aber auch möglich das Verfahren bei Unterdruck oder bei Überdruck durchzuführen (beispielsweise in einem Druckbereich von 0,5 bis 5 bar).

Im allgemeinen setzt man 1 bis 2 Mol, bevorzugt 1 bis 1,5 Mol Oxidationsmittel, bezogen auf 1 Mol N-Indolylethyl-sulfonamid (IIa) ein. Ganz besonders bevorzugt arbeitet man mit molaren Mengen der Reaktanden.

Das erfindungsgemäße Verfahren kann beispielsweise wie folgt durchgeführt werden:
Das N-Indolylethylsulfonamid wird in Dimethylsulfoxid, das gleichzeitig Verdünnungsmittel und Oxidationsmittel ist, suspendiert und mit Säure versetzt. Nach Beendigung der Reaktion wird durch Extraktion aufgearbeitet.

Die als Ausgangsstoffe eingesetzten N-Indolylethylsulfonamide der allgemeinen Formel (II) und deren Salze sind neu.
Sie können hergestellt werden, indem man Indolylethylamine der allgemeinen Formel (III)

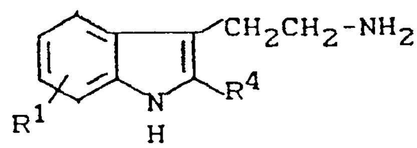

(III)

in welcher

- $R^1$ - für Wasserstoff, Fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino, Acetylamino steht, oder
  - für eine Gruppe der Formel $-OR^8$ steht,
  worin
  $R^8$ - Wasserstoff, $C_1$-$C_4$-Alkyl, Phenyl oder Benzyl bedeutet,

oder

- $R^1$ - für $C_1$-$C_4$-Alkyl steht,

und

- $R^4$ - für Wasserstoff steht

mit Sulfonsäurehalogeniden der allgemeinen Formel (IV)

$Y\text{-}SO_2\text{-}R^2$ (IV)

in welcher

- $R^2$ für Phenyl steht, das bis zu 2-fach gleich oder verschieden durch Fluor, Chlor, Brom, Cyano, Trifluormethyl, Trifluormethoxy, $C_1$-$C_4$-Alkoxy, Methylthio, Hydroxy, Methoxycarbonyl, Ethoxycarbonyl, Dimethylamino, Acetylamino, Diethylamino substituiert ist,

und

- Y - für Halogen steht,

und mit Acrylnitril der Formel (V)

$H_2C=CH\text{-}CN$ (V)

in Gegenwart eines inerten Lösungsmittels, gegebenenfalls in Gegenwart einer Base umsetzt, dann die N,N'-Biscyanoethylverbindungen verseift und im Fall der Herstellung der Salze mit einer entsprechenden Base umsetzt.

Die Herstellung der als Ausgangsstoffe verwendeten N-Indolylethyl-sulfonamide der Formeln (II) und (IIa) läßt sich durch folgendes Formelschema verdeutlichen:

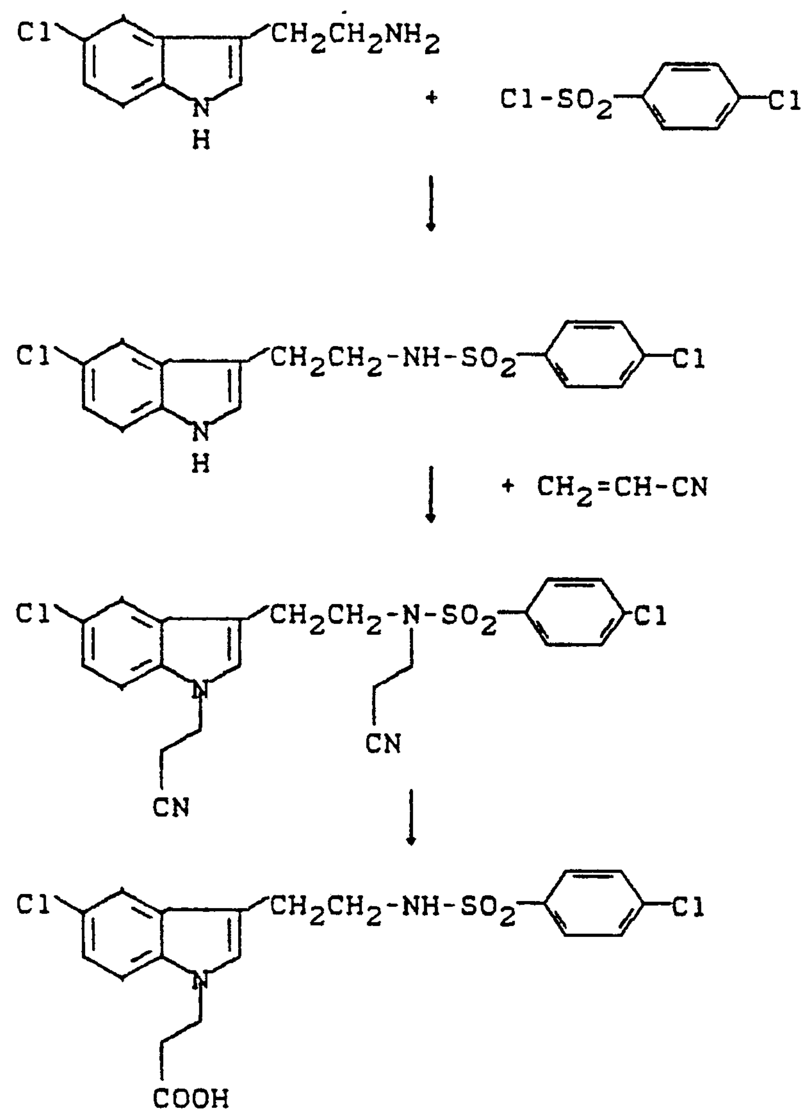


Bei der Durchführung des Verfahrens entstehen im allgemeinen Zwischenprodukte die isoliert werden können. So ist es möglich, das Verfahren in mehreren Verfahrensstufen auszuführen. Es kann aber auch möglich sein, verschiedene Verfahrensschritte zu kombinieren.

Die Indolylethylamine der allgemeinen Formel (III), die Sulfonsäurehalogenide der allgemeinen Formel (IV) und Acrylnitril der Formel (V) sind an sich bekannt und können nach an sich bekannten Methoden hergestellt werden (Houben-Weyls "Methoden der organischen Chemie" Bd. IX, 407 ff. und 547 ff. (1959); The Chemistry of Indols, Academic Press 1970; W.J. Honlihan, Indoles Part II, John Wiley & Sons, 1972).

Lösungsmittel für das Verfahren können inerte organische Lösungsmittel sein, die sich unter den Reaktionsbedingungen nicht verändern. Hierzu gehören bevorzugt Alkohole wie beispielsweise Methanol, Ethanol, Propanol oder Isopropanol, oder Ether wie beispielsweise Diethylether, Dioxan, Tetrahydrofuran, oder Halogenkohlenwasserstoffe wie Dichlormethan, Trichlormethan, Tetrachlormethan, Dichlorethylen oder Trichlorethylen, oder Essigsäure, Toluol, Acetonitril, Nitromethan, Dimethylformamid, Hexamethylphosphorsäuretriamid, Pyridin und Aceton. Selbstverständlich ist es möglich, Gemische der Lösungsmittel einzusetzen.

Als Basen können übliche basische Verbindungen für basische Reaktionen eingesetzt werden. Hierzu gehören vorzugsweise Alkali- und Erdalkalihydroxide oder carbonate wie beispielsweise Lithium-, Natrium-, Kalium-, Calcium- oder Bariumhydroxid, Natrium- oder Kaliumcarbonat, Alkalialkoholate wie beispielsweise Natriummethanolat und -ethanolat oder Kaliummethanolat oder -ethanolat, oder organische Basen wie beispielsweise Triethylamin, Pyridin oder 1-Methylpiperidin, Benzyltrimethylammomiumhydroxid oder Tetrabutylammoniumhydroxid. Das Verfahren wird im allgemeinen in Temperaturbereich von -20° C bis +100° C, bevorzugt von 0° C bis 80° C durchgeführt.

Das Verfahren wird im allgemeinen bei Normaldruck durchgeführt. Es ist aber auch möglich, das

Verfahren bei Unter- oder Überdruck durchzuführen (beispielsweise in einem Druckbereich von 0,5 bis 10 bar).

Im allgemeinen setzt man 1 bis 5 Mol, bevorzugt 1 bis 2 Mol, besonders bevorzugt 1 Mol Sulfonsäurehalogenid bezogen auf 1 Mol des Indolylethylamins ein.
Acrylnitril wird im allgemeinen in einer Menge von 1 bis 10 Mol, bevorzugt 1 bis 5 Mol, besonders bevorzugt 3 Mol bezogen auf 1 Mol des Indolylethylamins eingesetzt.

Die Verseifung wird im allgemeinen in Gegenwart von Basen, bevorzugt Alkali- oder Erdalkalihydroxiden oder alkoholaten durchgeführt. Vorzugsweise werden Basen wie Alkali- oder Erdalkalihydroxide oder Alkalialkoholate, bevorzugt Lithium-, Natrium-, Kalium-, Calcium- oder Bariumhydroxid, oder Natrium- bzw. Kaliummethylat oder -ethylat verwendet.

Im allgemeinen setzt man bei der Verseifung 1 bis 100 Mol, bevorzugt 2 bis 50 Mol der Base bezogen auf 1 Mol der N,N'-Biscyanoethylverbindung ein.

Als Indolyethylamine können beispielsweise erfindungsgemäß verwendet werden:
2-(5-Methyl-1H-indol-3-yl)ethylamin
2-(5-Methoxy-1H-indol-3-yl)ethylamin
2-(5-Fluor-1H-indol-3-yl)ethylamin
2-(5-Chlor-1H-indol-3-yl)ethylamin.

Als Sulfonsäurehalogenide können beispielsweise erfindungsgemäß verwendet werden:
4-Chlorphenylsulfonylchlorid
4-Fluorphenylsulfonylchlorid
4-Methylphenylsulfonylchlorid
4-Toluolsulfonylchlorid
4-Methoxyphenylsulfonylchlorid

Als N-Indolylethyl-sulfonamide können beispielsweise er findungsgemäß verwendet werden:
N-[2-[1-(2-Carboxyethyl)-1H-indol-3-yl]ethyl]benzolsulfonamid
N-[2-[1-(2-Carboxyethyl)-1H-indol-3-yl]ethyl]benzolsulfonamid-Natriumsalz
N-[2-[1-(2-Carboxyethyl)-1H-indol-3-yl]ethyl]benzolsulfonamid-Triethylammoniumsalz
N-[2-[1-(2-Carboxyethyl)-1H-indol-3-yl]ethyl]-(4-fluorphenyl)sulfonamid-Triethylammoniumsalz
N-[2-[1-(2-Carboxyethyl)-2-methyl-1H-indol-3-yl]ethyl]-(4-chlorphenyl)sulfonamid
N-[2-[1-(2-Carboxyethyl)-2-methyl-1H-indol-3-yl]ethyl]-(4-chlorphenyl)sulfonamid-Triethylammoniumsalz
N-[2-[1-(2-Carboxyethyl)-5-fluor-2-methyl-1H-indol-3-yl]ethyl]-(4-fluorphenyl)sulfonamid

Die neuen N-Dihydroindolylethyl-sulfonamide bzw. deren Salze können als Wirkstoffe in Arzneimitteln eingesetzt werden. Die Wirkstoffe weisen beispielsweise eine thrombozytenaggregationshemmende und Thromboxan $A_2$-antagonistische Wirkung auf. Sie können bevorzugt zur Behandlung von Thrombosen, Thromboembolien, Ischämien, als Antiasthmatika und als Antiallergika eingesetzt werden.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Waser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyehtylen-Fettsäure-Ester), Polyoxyethylen-Fettalkohol-Ether, Alkylsulfonate, Arylsulfonate (z.B. Lignin Sulfitablaugen, Methylcellulose, Stärke und Polyvinylpyrrolidon) und Gleitmittel (z.B.Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten, Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten

Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Herstellungsbeispiele

### Beispiel 1

N-[2-[1-(2-Carboxyethyl)-2,3-dihydro-1H-indol-3-yl]-ethyl]benzolsulfonamid

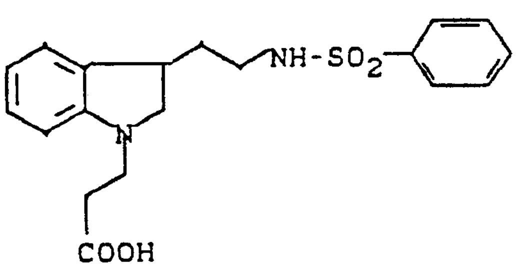

0,95 g (2 mmol) N-[2-[1-(2-Carboxyethyl)-1H-indol-3-yl]-ethyl]benzolsulfonamid-Triethylammoniumsalz werden bei Raumtemperatur in 10 ml Trifluoressigsäure gelöst und mit 0,23 g (2 mmol) Triethylsilan versetzt. Nach 10 stündigem Stehen wird mit 2 n Natronlauge verdünnt und mit Essigester extrahiert. Die wäßrige Phase wird mit 2 n Schwefelsäure sauer gestellt und zweimal mit Essigester extrahiert. Die vereinigten Essigesterphasen werden mit Natriumsulfat getrocknet und eingedampft. Der Rückstand wird nach dem Trocknen im Hochvakuum an 100 g Kieselgel (Merck, 0,63 -0,2 mm) mit einem Gemisch von Methylenchlorid und Methanol im Verhältnis von 96 zu 4 gereinigt. Man erhält so eine Fraktion, die 0,68 g zähes, öliges Produkt enthält ($R_f$ = 0,12, $CH_2Cl_2$ : Essigester = 100:2).
Zur Herstellung des Hydrochlorids wird das Produkt in Aceton gelöst und mit 3 ml 1 n Salzsäure versetzt. Nach gründlichem Eindampfen im Vakuum erhält man 0,7 g (85% der Theorie) festen Rückstand.
Schmelzpunkt: 70-80 °C

### Beispiel 2

N-[2-[1-(2-Carboxyethyl)-2,3-dihydro-1H-indol-3-yl]-ethyl]-(4-fluorphenyl)sulfonamid

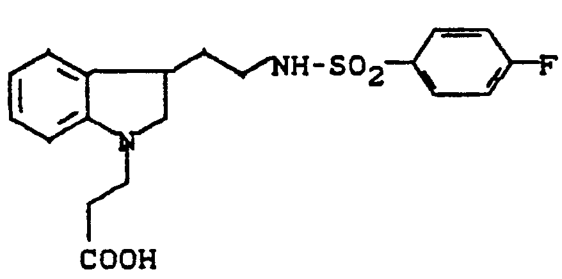

0,75 g (1,9 mmol) N-[2-[1-(2-Carboxyethyl)-1H-indol-3-yl]ethyl]-(4-fluorphenyl)sulfonamid werden analog zu Beispiel 1 mit Triethylsilan reduziert. Man erhält so 0,58 g zähes, öliges Produkt ($R_f$ = 0,18, $CH_2Cl_2$ : Essigester = 100:2). Nach Umwandlung des Produktes in sein Hydrochlorid (analog Beispiel 1) erhält man 0,62 g (76% der Theorie) festen Rückstand.
Schmelzpunkt: 70-80° C

Beispiel 3

N-[2-[1-(2-Carboxyethyl)-2,3-dihydro-2-methyl-1H-indol-3-yl]ethyl]-(4-chlorphenyl)sulfonamid

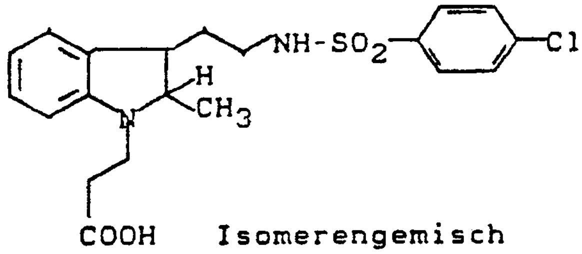

Isomerengemisch

0,9 g (2,1 mmol) N-[2-[1-(2-Carboxyethyl)-2-methyl-1H-indol-3-yl]ethyl]-(4-chlorphenyl)sulfonamid werden in 10 ml Trifluoressigesäure bei 0° C portionsweise mit 1 g Natriumcyanoborhydrid versetzt. Nach 2 stündigem Rühren bei 0 bis 5° C wird die Reaktionslösung mit Essigester verdünnt und dreimal mit ges. Kochsalzlösung extrahiert. Die Essigesterphase wird mit Natriumsulfat getrocknet und im Vakuum gründlich eingedampft. Der Rückstand wird an Kieselgel (Merck, 0,04 bis 0,063 mm) mit einem Gemisch von Methylenchlorid und Methanol im Verhältnis 96 zu 4 chromatographiert. Man erhält so eine Fraktion, die nach dem Eindampfen 0,8 g zähes, öliges Produkt ergibt ($R_f$ = 0,3, $CH_2Cl_2$ : Eisessig = 100:2). Dieses zähe Öl wird analog zu Beispiel 1 in sein Hydrochlorid umgewandelt: 0,82 g (85% der Theorie) fester Rückstand.
Schmelzpunkt: 75-80° C

Beispiel 4

N-[2-[1-(2-Carboxyethyl)-2,3-dihydro-2-methyl-1H-indol-3-yl]ethyl]-(4-fluorphenyl)sulfonamid

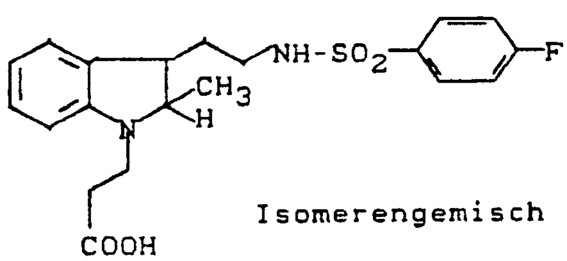

Isomerengemisch

1 g (2,5 mmol) N-[2-[1-(2-Carboxyethyl)-2-methyl-1H-indol-3-yl]ethyl]-(4-fluorphenyl)sulfonamid werden analog zu Beispiel 3 mit Natriumcyanoborhydrid reduziert. Man erhält so 0,85 g zähes, öliges Produkt ( $R_f$ = 0,32, $CH_2Cl_2$ : Eisessig = 100:2), das analog zu Beispiel 1 in sein Hydrochlorid umgewandelt wird: 0,9 g (81% der Theorie) fester Rückstand.
Schmelzpunkt: 75-80° C

Beispiel 5 und Beispiel 6

N-[2-[1-(2-Carboxyethyl)-5-fluor-2,3-r-dihydro-2-c-methyl-1H-indol-3-yl]ethyl]-(4-fluorphenyl)sulfonamid

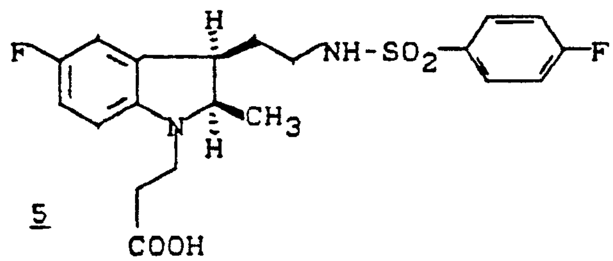

5

und N-[2-[1-(2-Carboxyethyl)-5-fluor-2,3-r-dihydro-2-t-methyl-1H-indol-3-yl]ethyl]-(4-fluorphenyl)sulfonamid

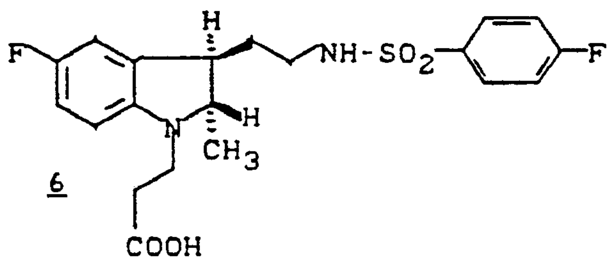

6

5,47 g (0,013 Mol) N-[2-[1-(2-Carboxyethyl)-5-fluor-2-methyl-1H-indol-3-yl]ethyl]-(4-fluorphenyl)-sulfonamid werden analog zu Beispiel 3 mit Natriumcyanoborhydrid reduziert. Nach Säulenchromatographie an 400 g Kieselgel (Merck, 0,04 - 0,063 mm) mit einem 100:1-Gemisch von Methylenchlorid mit Eisessig erhält man zwei Fraktionen, von denen die erste nach Eindampfen 0,47 g Isomer A ergibt ($R_f$ = 0,5, $CH_2Cl_2$ : Eisessig = 100:2). Die zweite Fraktion ergibt nach Eindampfen 2,28 g Isomer B ($R_f$ = 0,42 g, $CH_2Cl_2$ : Eisessig = 100:2). Die so erhaltenen Isomere A und B werden analog zu Beispiel 1 in ihre Hydrochloride umgewandelt. Dabei erhält man 2,35 g (39% der Theorie) festes Hydrochlorid von Isomer B und 0,53 g (9 % der Theorie) festes Hydrochlorid von Isomer A.
Schmelzpunkt von Isomer A: 80-90°C
Schmelzpunkt von Isomer B: 58-70°C

Beispiel 7

N-[2-[1-(2-Carboxyethyl)-2,3-dihydro-2-oxo-1H-indol-3-yl]ethyl]benzolsulfonamid

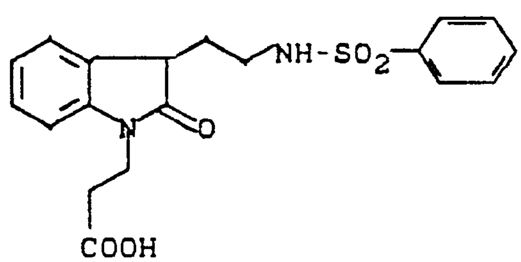

0,74 g (1,56 mmol) N-[2-[1-(2-Carboxyethyl)-1H-indol-3-yl]ethyl]benzolsulfonamid-Triethylammoniumsalz werden in 8 ml Dimethylsulfoxid suspendiert und mit 4 ml konz. Salzsäure versetzt. Die klare Lösung wird 3 h bei 50°C gerührt. Anschließend wird mit Wasser verdünnt und mit Essigester extrahiert. Die Essigesterphase wird noch zweimal mit Wasser gewaschen, mit Natriumsulfat getrocknet und eingedampft. Man erhält so 0,57 g (94% der Theorie) festes Produkt.
$R_f$ = 0,34 , $CH_2Cl_2$ : $CH_3OH$ = 9:1
Schmelzpunkt: 50-60°C

Beispiel 8

N-[2-[1-(2-Carboxyethyl)-2,3-dihydro-2-oxo-1H-indol-3-yl]ethyl]-(4-chlorphenyl)sulfonamid

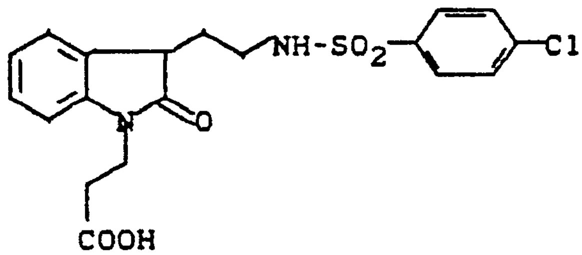

1,72 g (3,4 mmol) N-[2-[1-(2-Carboxyethyl)-1H-indol-3-yl]ethyl]-(4-chlorphenyl)sulfonamid-Triethylammoniumsalz werden analog zu Beispiel 7 oxidiert. Man erhält so 1,4 g (97% der Theorie) festes Produkt.
$R_f$ = 0,44, $CH_2Cl_2$ : $CH_3OH$ = 9:1
Schmelzpunkt: 60-70° C

Beispiel 9

N-[2-[1-(2-Carboxyethyl)-2,3-dihydro-2-oxo-1H-indol-3-yl]ethyl]-(4-fluorphenyl)sulfonamid

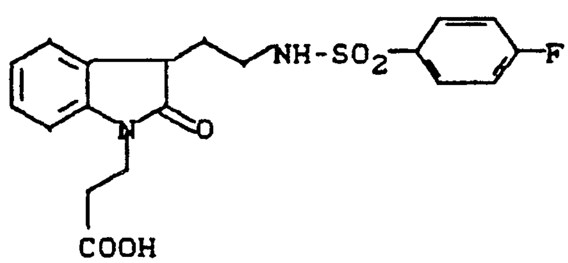

3 g (7,7 mmol) N-[2-[1-(2-Carboxyethyl)-1H-indol-3-yl]ethyl-(4-fluorphenyl)sulfonamid werden analog zu Beispiel 7 oxidiert. Man erhält so 3 g (96% der Theorie) festes Produkt.
$R_f$ = 0,36, $CH_2Cl_2$ : $CH_3OH$ = 9:1
Schmelzpunkt: 60-70° C

Beispiel 10
Anwendungsbeispiel

Zur Bestimmung der thrombozytenaggregationshemmenden Wirkung wurde Blut von gesunden Probanden beiderlei Geschlechts verwendet. Als Antikoagulans wurde einem Teil 3,8%iger wäß riger Natriumzitratlösung 9 Teile Blut zugemischt. Mittels Zentrifugation erhält man aus diesem Blut plättchenreiches Zitratplasma (PRP) (Jürgens/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37° C im Wasserbad vorinkubiert. Anschließend wurde die Thrombozytenaggregation nach der turbidometrischen Methode (Born, G.V.R., J. Physiol. (London ), 162, 67, 1962) im Aggregometer bei 37° C bestimmt (Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe der PRP wurde während einer Zeitdauer von 6 Minuten aufgezeichnet und der Ausschlag nach 6 Minuten bestimmt. Hierzu wird die prozentuale Hemmung gegenüber der Kontrolle errechnet.

| N-Dihydroindolylethyl-sulfonamide nach Beispiel-Nr. | Grenzkonzentration für Hemmung (mg/l) |
|---|---|
| 1 | 10 bis 3,0 |
| 2 | 1 bis 0,3 |
| 3 | 1 bis 0,3 |
| 4 | 0,3 bis 0,1 |
| 5 | 0,1 bis 0,01 |
| 7 | 1 bis 0,1 |
| 8 | 0,1 bis 0,03 |

## Patentansprüche

**Patentansprüche für folgende Vertragsstaaten : AT, BE, CH, DE, FR, GB, IT, LI, NL, SE**

**1.** N-Dihydroindolylethyl-sulfonamide der allgemeinen Formel

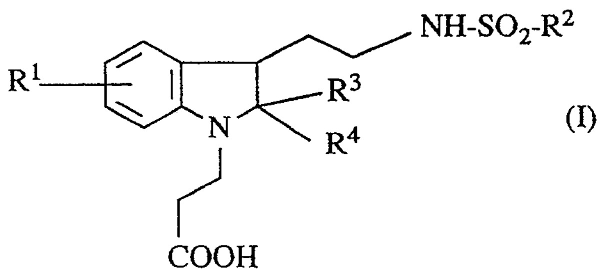

(I)

worin

$R^1$ - für Wasserstoff, Fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino, Acetylamino steht, oder

- für eine Gruppe der Formel $-OR^8$ steht,

worin

$R^8$ - Wasserstoff, $C_1$-$C_4$-Alkyl, Phenyl oder Benzyl bedeutet,

oder

$R^1$ - für $C_1$-$C_4$-Alkyl steht,

$R^2$ - für Phenyl steht, das bis zu 2-fach gleich oder verschieden durch Fluor, Chlor, Brom, Cyano, Trifluormethyl, Trifluormethoxy, $C_1$-$C_4$-Alkoxy, Methylthio, Hydroxy, Methoxycarbonyl, Ethoxycarbonyl, Dimethylamino, Acetylamino, Diethylamino substituiert ist,

$R^3$ - für Wasserstoff oder $C_1$-$C_4$-Alkyl,

und

$R^4$ - für Wasserstoff steht,

oder

$R^3$ und $R^4$ gemeinsam einen Carbonylsauerstoff binden,

und deren Salze.

**2.** N-Dihydroindolylethyl-sulfonamide der Formel nach Anspruch 1

worin

$R^1$ - für Wasserstoff, Fluor, Chlor oder Brom steht,

$R^2$ - für Phenyl steht, das durch Flour, Chlor oder Brom substituiert ist,
$R^3$ - für Wasserstoff oder $C_1$-$C_4$-Alkyl,
und
$R^4$ - für Wasserstoff steht,
oder
$R^3$ und $R^4$ gemeinsam einen Carbonylsauerstoff binden,
und deren Salze.

3. N-Dihydroindolylethyl-sulfonamide nach Anspruch 1 zur therapeutischen Anwendung.

4. Verfahren zur Herstellung von N-Dihydroindolylethyl-sulfonamiden der allgemeinen Formel

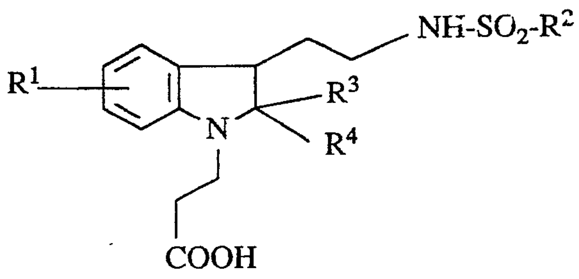

(Ia)

worin
$R^1$ - für Wasserstoff, Fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino, Acetylamino steht, oder
- für eine Gruppe der Formel -$OR^8$ steht,
worin
$R^8$ - Wasserstoff, $C_1$-$C_4$-Alkyl, Phenyl oder Benzyl bedeutet,
oder
$R^1$ - für $C_1$-$C_4$-Alkyl steht,
$R^2$ - für Phenyl steht, das bis zu 2-fach gleich oder verschieden durch Fluor, Chlor, Brom, Cyano, Trifluormethyl, Trifluormethoxy, $C_1$-$C_4$-Alkoxy, Methylthio, Hydroxy, Methoxycarbonyl, Ethoxycarbonyl, Dimethylamino, Acetylamino, Diethylamino substituiert ist,
$R^3$ - für Wasserstoff oder $C_1$-$C_4$-Alkyl,
und
$R^4$ - für Wasserstoff steht,
und deren Salze, dadurch gekennzeichnet, daß man N-Indolylethyl-sulfonamide der allgemeinen Formel (II)

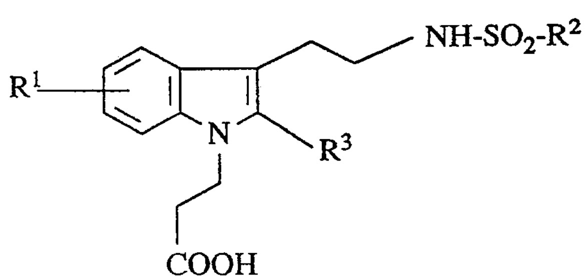

(II)

in welcher
$R^1$, $R^2$ und $R^3$ die angegebene Bedeutung haben, oder deren Salze, gegebenenfalls in Gegenwart eines inerten organischen Lösungsmittels in Anwesenheit einer Säure und eines Reduktionsmittels hydriert, gegebenenfalls Isomeren in üblicher Weise trennt und im Fall der Herstellung der Salze mit einer entsprechenden Base umsetzt.

5. Verfahren zur Herstellung von N-Dihydroindolylethyl-sulfonamiden der allgemeinen Formel

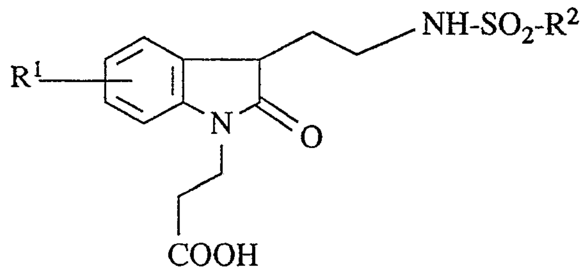

(Ib)

in welcher

$R^1$ - für Wasserstoff, Fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino, Acetylamino steht, oder
- für eine Gruppe der Formel $-OR^8$ steht,
worin
$R^8$ - Wasserstoff, $C_1$-$C_4$-Alkyl, Phenyl oder Benzyl bedeutet,

oder

$R^1$ - für $C_1$-$C_4$-Alkyl steht,

$R^2$ - für Phenyl steht, das bis zu 2-fach gleich oder verschieden durch Fluor, Chlor, Brom, Cyano, Trifluormethyl, Trifluormethoxy, $C_1$-$C_4$-Alkoxy, Methylthio, Hydroxy, Methoxycarbonyl, Ethoxycarbonyl, Dimethylamino, Acetylamino, Diethylamino substituiert ist,

und deren Salze,

dadurch gekennzeichnet, daß man N-Indolylethyl-sulfonamide der allgemeinen Formel

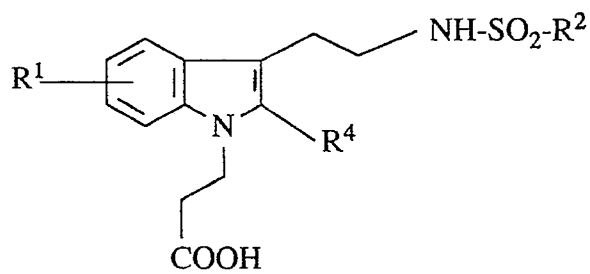

(IIa)

in welcher

$R^1$ und $R^2$ die angegebene Bedeutung haben und

$R^4$ - Wasserstoff steht,

oder deren Salze,

in inertem Lösemittel, gegebenenfalls in Anwesenheit einer Säure oxidiert und im Fall der Herstellung der Salze mit einer entsprechenden Base umsetzt.

6. Arzneimittel enthaltend mindestens ein N-Dihydroindolylethyl-sulfonamid nach Anspruch 1.

7. Arzneimittel nach Anspruch 6, enthaltend 0,5 bis 90 Gew.-% N-Dihydroindolylethylsulfonamid bezogen auf die Gesamtmischung.

8. Verfahren zur Herstellung von Arzneimitteln nach Anspruch 6, dadurch gekennzeichnet, daß man das N-Dihydroindolylethyl-sulfonamid gegebenenfalls mit Hilfe von üblichen Hilfs- und Trägerstoffen in eine geeignete Applikationsform bringt.

9. Verwendung der N-Dihydroindolylethyl-sulfonamide nach Anspruch 1 zur Herstellung von Arzneimitteln.

10. Verwendung der N-Dihydroindolylethyl-sulfonamide nach Anspruch 1 zur Herstellung von Arzneimitteln

zur Behandlung von Thrombosen, Thromoembolien, Ischämien und asthmatischen und allergischen Erkrankungen.

**Patentansprüche für folgenden Vertragsstaat : ES**

1. Verfahren zur Herstellung von N-Dihydroindolylethyl-sulfonamiden der allgemeinen Formel

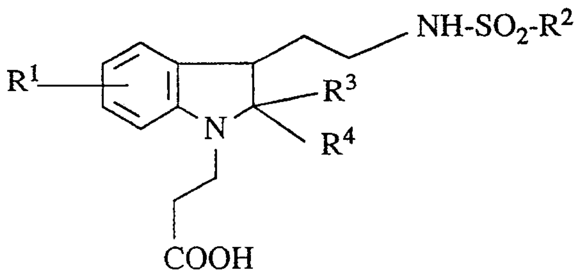

(Ia)

worin

$R^1$ - für Wasserstoff, Fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino, Acetylamino steht, oder
- für eine Gruppe der Formel $-OR^8$ steht,
worin
$R^8$ - Wasserstoff, $C_1$-$C_4$-Alkyl, Phenyl oder Benzyl bedeutet,

oder

$R^1$ - für $C_1$-$C_4$-Alkyl steht,

$R^2$ - für Phenyl steht, das bis zu 2-fach gleich oder verschieden durch Fluor, Chlor, Brom, Cyano, Trifluormethyl, Trifluormethoxy, $C_1$-$C_4$-Alkoxy, Methylthio, Hydroxy, Methoxycarbonyl, Ethoxycarbonyl, Dimethylamino, Acetylamino, Diethylamino substituiert ist,

$R^3$ - für Wasserstoff oder $C_1$-$C_4$-Alkyl,

und

$R^4$ - für Wasserstoff steht,

und deren Salze, dadurch gekennzeichnet, daß man N-Indolylethyl-sulfonamide der allgemeinen Formel (II)

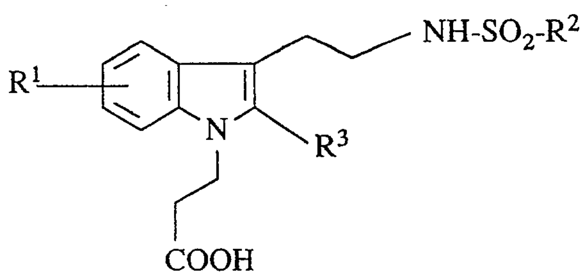

(II)

in welcher

$R^1$, $R^2$ und $R^3$ die angegebene Bedeutung haben, oder deren Salze, gegebenenfalls in Gegenwart eines inerten organischen Lösungsmittels in Anwesenheit einer Säure und eines Reduktionsmittels hydriert, gegebenenfalls Isomeren in üblicher Weise trennt und im Fall der Herstellung der Salze mit einer entsprechenden Base umsetzt.

2. Verfahren nach Anspruch 1, dadurch gekennzeichnet, daß man als Säure Carbonsäuren oder Sulfonsäuren einsetzt.

3. Verfahren nach Anspruch 1, dadurch gekennzeichnet, daß man als Reduktionsmittel Hydride einsetzt.

4. Verfahren nach Anspruch 1, dadurch gekennzeichnet, daß man in einem Temperaturbereich von -40°C bis +80°C arbeitet.

5. Verfahren nach Anspruch 1, dadurch gekennzeichnet, daß man in einem Temperaturbereich von -20°C bis +60°C arbeitet.

6. Verfahren zur Herstellung von N-Dihydroindolylethyl-sulfonamiden der allgemeinen Formel

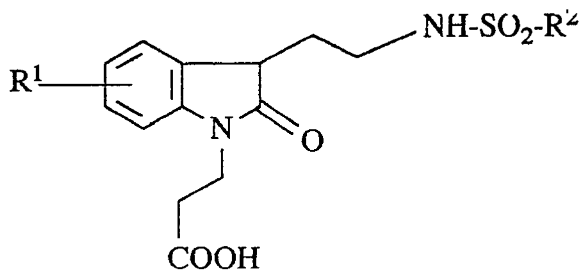

(Ib)

in welcher

$R^1$ - für Wasserstoff, Fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino, Acetylamino steht, oder
- für eine Gruppe der Formel $-OR^8$ steht,
worin
$R^8$ - Wasserstoff, $C_1$-$C_4$-Alkyl, Phenyl oder Benzyl bedeutet,

oder

$R^1$ - für $C_1$-$C_4$-Alkyl steht,
$R^2$ - für Phenyl steht, das bis zu 2-fach gleich oder verschieden durch Fluor, Chlor, Brom, Cyano, Trifluormethyl, Trifluormethoxy, $C_1$-$C_4$-Alkoxy, Methylthio, Hydroxy, Methoxycarbonyl, Ethoxycarbonyl, Dimethylamino, Acetylamino, Diethylamino substituiert ist,

und deren Salze,

dadurch gekennzeichnet, daß man N-Indolylethyl-sulfonamide der allgemeinen Formel

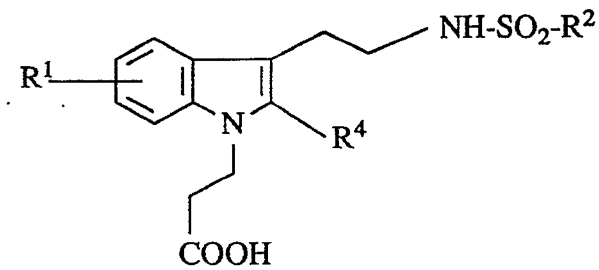

(IIa)

in welcher

$R^1$ und $R^2$ die angegebene Bedeutung haben und
$R^4$ - Wasserstoff steht,

oder deren Salze,

in inertem Lösemittel, gegebenenfalls in Anwesenheit einer Säure oxidiert und im Fall der Herstellung der Salze mit einer entsprechenden Base umsetzt.

7. Verfahren nach Anspruch 6, dadurch gekennzeichnet, daß man als Oxidationsmittel N-Bromsuccinimidhalogene oder Dimethylsulfoxid einsetzt.

8. Verfahren nach Anspruch 6, dadurch gekennzeichnet, daß man als Säure Salzsäure, Bromwasserstoffsäure, Iodwasserstoffsäure, Schwefelsäure, Phosphorsäure, Essigsäure, Trifluoressigsäure, Chloressigsäure, Dichloressigsäure, Trichloressigsäure, Methansulfonsäure, Ethansulfonsäure, Benzolsulfonsäure,

Toluolsulfonsäure oder Trifluormethansulfonsäure einsetzt.

9. Verfahren nach Anspruch 6, dadurch gekennzeichnet, daß man die Oxidation in einem Temperaturbereich von $0^\circ$ C bis $+100^\circ$ C durchführt.

10. Verfahren nach Anspruch 6, dadurch gekennzeichnet, daß man die Oxidation in einem Temperaturbereich von $+20^\circ$ C bis $+60^\circ$ C durchführt.

**Claims**

**Claims for the following Contracting States : AT, BE, CH, DE, FR, GB, IT, LI, NL, SE**

1. N-Dihydroindolylethyl-sulphonamides of the general formula

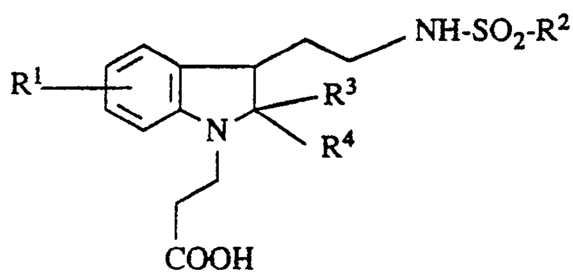

(I)

in which

$R^1$ - represents hydrogen, fluorine, chlorine, bromine, trifluoromethyl, methylthio, ethylthio, methylsulphonyl, ethylsulphonyl, phenylthio, phenylsulphonyl, amino, dimethylamino, diethylamino or acetylamino, or
- represents a group of the formula $-OR^8$,
in which
$R^8$ - denotes hydrogen, $C_1$-$C_4$-alkyl, phenyl or benzyl,

or

$R^1$ - represents $C_1$-$C_4$-alkyl,

$R^2$ - represents phenyl which is monosubstituted or disubstituted by identical or different substituents from the series comprising fluorine, chlorine, bromine, cyano, trifluoromethyl, trifluoromethoxy, $C_1$-$C_4$-alkoxy, methylthio, hydroxyl, methoxycarbonyl, ethoxycarbonyl, dimethylamino, acetylamino and diethylamino,

$R^3$ - represents hydrogen or $C_1$-$C_4$-alkyl,

and

$R^4$ - represents hydrogen,

or $R^3$ and $R^4$ together form a carbonyl oxygen,
and their salts.

2. N-Dihydroindolylethyl-sulphonamides of the formula according to Claim 1
in which

$R^1$ - represents hydrogen, fluorine, chlorine or bromine,

$R^2$ - represents phenyl which is substituted by fluorine, chlorine or bromine,

$R^3$ - represents hydrogen or $C_1$-$C_4$-alkyl,

and

$R^4$ - represents hydrogen,

or

$R^3$ and $R^4$ together form a carbonyl oxygen,
and their salts.

3. N-Dihydroindolylethyl-sulphonamides according to Claim 1 for therapeutic use.

4. Process for the preparation of N-dihydroindolylethyl-sulphonamides of the general formula

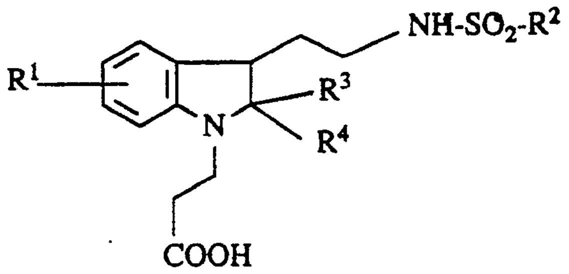

(Ia)

in which

$R^1$ - represents hydrogen, fluorine, chlorine, bromine, trifluoromethyl, methylthio, ethylthio, methylsulphonyl, ethylsulphonyl, phenylthio, phenylsulphonyl, amino, dimethylamino, diethylamino or acetylamino, or
- represents a group of the formula $-OR^8$,
in which
$R^8$ - denotes hydrogen, $C_1$-$C_4$-alkyl, phenyl or benzyl,

or

$R^1$ - represents $C_1$-$C_4$-alkyl,

$R^2$ - represents phenyl which is monosubstituted or disubstituted by identical or different substituents from the series comprising fluorine, chlorine, bromine, cyano, trifluoromethyl, trifluoromethoxy, $C_1$-$C_4$-alkoxy, methylthio, hydroxyl, methoxycarbonyl, ethoxycarbonyl, dimethylamino, acetylamino and diethylamino,

$R^3$ - represents hydrogen or $C_1$-$C_4$-alkyl,

and

$R^4$ - represents hydrogen,

and their salts, characterised in that N-indolylethylsulphonamides of the general formula (II)

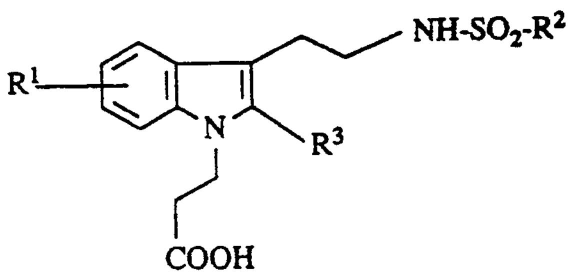

(II)

in which

$R^1$, $R^2$ and $R^3$ have the meaning indicated, or their salts, are hydrogenated in the presence of an acid and a reducing agent, if appropriate in the presence of an inert organic solvent, if appropriate isomers are separated in a customary manner and in the case of the preparation of the salts reacted with an appropriate base.

**5.** Process for the preparation of N-dihydroindolylethyl-sulphonamides of the general formula

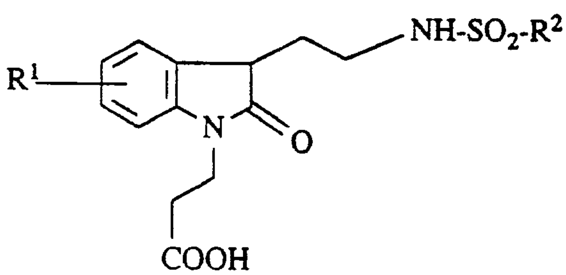

(Ib)

in which

$R^1$ - represents hydrogen, fluorine, chlorine, bromine, trifluoromethyl, methylthio, ethylthio, methylsulphonyl, ethylsulphonyl, phenylthio, phenylsulphonyl, amino, dimethylamino, diethylamino or acetylamino, or

- represents a group of the formula $-OR^8$,

in which

$R^8$ - denotes hydrogen, $C_1$-$C_4$-alkyl, phenyl or benzyl,

or

$R^1$ - represents $C_1$-$C_4$-alkyl,

$R^2$ - represents phenyl which is monosubstituted or disubstituted by identical or different substituents from the series comprising fluorine, chlorine, bromine, cyano, trifluoromethyl, trifluoromethoxy, $C_1$-$C_4$-alkoxy, methylthio, hydroxyl, methoxycarbonyl, ethoxycarbonyl, dimethylamino, acetylamino and diethylamino,

and their salts,

characterised in that N-indolylethyl-sulphonamides of the general formula

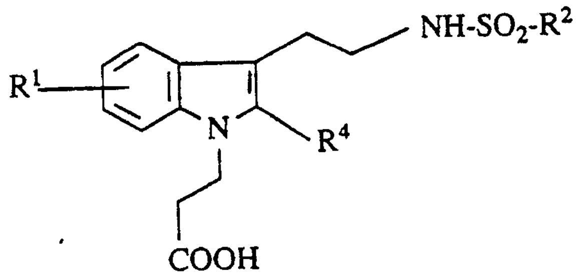

(IIa)

in which

$R^1$ and $R^2$ have the meaning indicated and

$R^4$ - represents hydrogen,

or their salts,

are oxidised in inert solvent, if appropriate in the presence of an acid and, in the case of the preparation of the salts, reacted with an appropriate base.

6. Medicaments containing at least one N-dihydroindolylethyl-sulphonamide according to Claim 1.

7. Medicaments according to Claim 6, containing 0.5 to 90 % by weight of N-dihydroindolylethyl-sulphonamide relative to the total mixture.

8. Process for the production of medicaments according to Claim 6, characterised in that the N-dihydroindolylethyl-sulphonamide is brought into a suitable administration form, if appropriate with the aid of customary auxiliaries and excipients.

9. Use of the N-dihydroindolylethyl-sulphonamides according to Claim 1 for the production of medicaments.

10. Use of the N-dihydroindolylethyl-sulphonamides according to Claim 1 for the production of medicaments for the treatment of thromboses, thromboembolisms, ischaemias and asthmatic and allergic diseases.

**Claims for the following Contracting State : ES**

1. Process for the preparation of N-dihydroindolylethyl-sulphonamides of the general formula

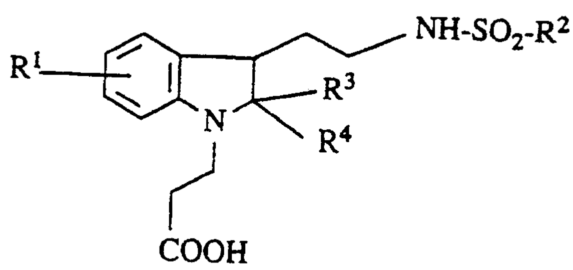

(Ia)

in which

$R^1$ - represents hydrogen, fluorine, chlorine, bromine, trifluoromethyl, methylthio, ethylthio, methylsulphonyl, ethylsulphonyl, phenylthio, phenylsulphonyl, amino, dimethylamino, diethylamino or acetylamino, or

- represents a group of the formula $-OR^8$,

in which

$R^8$ - denotes hydrogen, $C_1$-$C_4$-alkyl, phenyl or benzyl,

or

$R^1$ - represents $C_1$-$C_4$-alkyl,

$R^2$ - represents phenyl which is monosubstituted or disubstituted by identical or different substituents from the series comprising fluorine, chlorine, bromine, cyano, trifluoromethyl, trifluoromethoxy, $C_1$-$C_4$-alkoxy, methylthio, hydroxyl, methoxycarbonyl, ethoxycarbonyl, dimethylamino, acetylamino and diethylamino,

$R^3$ - represents hydrogen or $C_1$-$C_4$-alkyl,

and

$R^4$ - represents hydrogen,

and their salts, characterised in that N-indolylethylsulphonamides of the general formula (II)

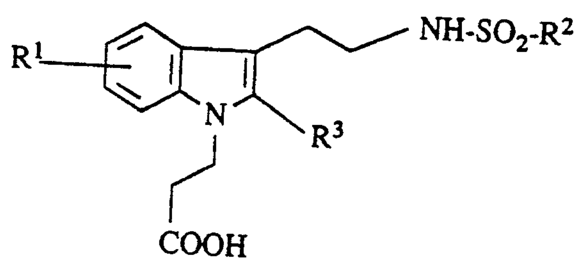

(II)

in which

$R^1$, $R^2$ and $R^3$ have the meaning indicated, or their salts, are hydrogenated in the presence of an acid and a reducing agent, if appropriate in the presence of an inert organic solvent, if appropriate isomers are separated in a customary manner and in the case of the preparation of the salts reacted with an appropriate base.

2. Process according to Claim 1, characterised in that carboxylic acids or sulphonic acids are employed as the acid.

3. Process according to Claim 1, characterised in that hydrides are employed as the reducing agent.

4. Process according to Claim 1, characterised in that the reaction is carried out in a temperature range from -40 °C to +80 °C.

5. Process according to Claim 1, characterised in that the reaction is carried out in a temperature range from -20 °C to +60 °C.

6. Process for the preparation of N-dihydroindolylethyl-sulphonamides of the general formula

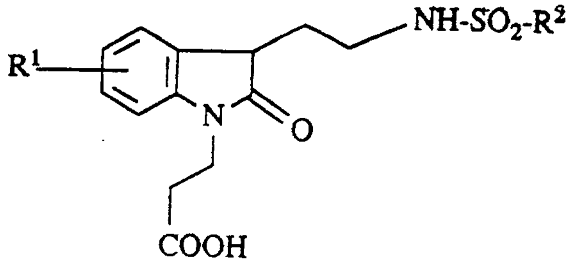

(Ib)

in which

$R^1$ - represents hydrogen, fluorine, chlorine, bromine, trifluoromethyl, methylthio, ethylthio, methylsulphonyl, ethylsulphonyl, phenylthio, phenylsulphonyl, amino, dimethylamino, diethylamino or acetylamino, or
- represents a group of the formula $-OR^8$,
in which
$R^8$ - denotes hydrogen, $C_1$-$C_4$-alkyl, phenyl or benzyl,

or

$R^1$ - represents $C_1$-$C_4$-alkyl,

$R^2$ - represents phenyl which is monosubstituted or disubstituted by identical or different substituents from the series comprising fluorine, chlorine, bromine, cyano, trifluoromethyl, trifluoromethoxy, $C_1$-$C_4$-alkoxy, methylthio, hydroxyl, methoxycarbonyl, ethoxycarbonyl, dimethylamino, acetylamino and diethylamino,

and their salts,
characterised in that N-indolylethyl-sulphonamides of the general formula

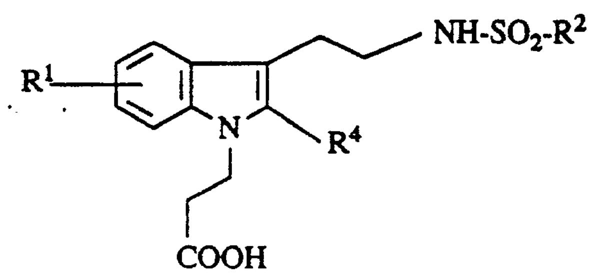

(IIa)

in which

$R^1$ and $R^2$ have the meaning indicated and

$R^4$ - represents hydrogen,

or their salts,
are oxidised in inert solvent, if appropriate in the presence of an acid and, in the case of the preparation of the salts, reacted with an appropriate base.

7. Process according to Claim 6, characterised in that N-bromosuccinimide, halogens or dimethyl sulphoxide is/are employed as the oxidising agent.

8. Process according to Claim 6, characterised in that hydrochloric acid, hydrobromic acid, hydriodic acid, sulphuric acid, phosphoric acid, acetic acid, trifluoroacetic acid, chloroacetic acid, dichloroacetic acid, trichloroacetic acid, methanesulphonic acid, ethanesulphonic acid, benzenesulphonic acid, toluenesulphonic acid or trifluoromethanesulphonic acid is employed as the acid.

9. Process according to Claim 6, characterised in that the oxidation is carried out in a temperature range from 0° C to +100° C.

10. Process according to Claim 6, characterised in that the oxidation is carried out in a temperature range from +20° C to +60° C.

**Revendications**

**Revendications pour les Etats contractants suivants : AT, BE, CH, DE, FR, GB, IT, LI, NL, SE**

1. N-dihydroindolyléthyl-sulfonamides de formule générale (I)

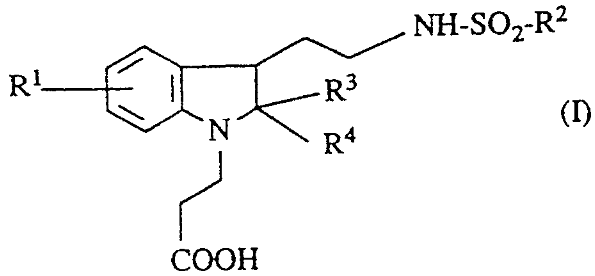

(I)

dans laquelle :

$R^1$ représente un atome d'hydrogène, un atome de fluor, un atome de chlore, un atome de brome, un groupe trifluorométhyle, un groupe méthylthio, un groupe éthylthio, un groupe méthylsulfonyle, un groupe éthylsulfonyle, un groupe phénylthio, un groupe phénylsulfonyle, un groupe amino, un groupe diméthylamino, un groupe diéthylamino, un groupe acétylamino ou
un groupe de formule $-OR^8$,
où
$R^8$ représente un atome d'hydrogène, un groupe alkyle en $C_1$-$C_4$, un groupe phényle ou un groupe benzyle

ou

$R^1$ un groupe alkyle en $C_1$-$C_4$,

$R^2$ représente un groupe phényle substitué jusqu'à deux fois, de manière identique ou différente, par un atome de fluor, un atome de chlore, un atome de brome, un groupe cyano, un groupe trifluorométhyle, un groupe trifluorométhoxy, un groupe alcoxy en $C_1$-$C_4$, un groupe méthylthio, un groupe hydroxyle, un groupe méthoxycarbonyle, un groupe éthoxycarbonyle, un groupe diméthylamino, un groupe acétylamino, un groupe diéthylamino,

$R^3$ représente un atome d'hydrogène ou un groupe alkyle en $C_1$-$C_4$,

et

$R^4$ représente un atome d'hydrogène

ou

$R^3$ et $R^4$ ensemble lient un atome d'oxygène carbonylique,

ainsi que leurs sels.

2. N-dihydroindolyléthyl-sulfonamides répondant à la formule selon la revendication 1
dans laquelle

$R^1$ représente un atome d'hydrogène, un atome de fluor, un atome de chlore ou un atome de brome,

$R^2$ représente un groupe phényle substitué par un atome de fluor, un atome de chlore ou un atome de brome,

$R^3$ représente un atome d'hydrogène ou un groupe alkyle en $C_1$-$C_4$,

et

$R^4$ représente un atome d'hydrogène

ou

$R^3$ et $R^4$ ensemble lient un atome d'oxygène carbonylique,

ainsi que leurs sels.

3. N-dihydroindolyléthyl-sulfonamides selon la revendication 1 pour l'application therapeutique.

4. Procédé pour la préparation de N-dihydroindolyléthyl-sulfonamides de formule générale

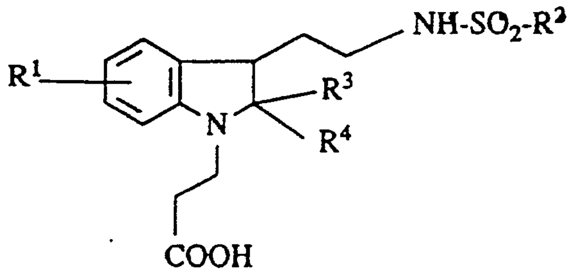

(Ia)

dans laquelle :

$R^1$ représente un atome d'hydrogène, un atome de fluor, un atome de chlore, un atome de brome, un groupe trifluorométhyle, un groupe méthylthio, un groupe éthylthio, un groupe méthylsulfonyle, un groupe éthylsulfonyle, un groupe phénylthio, un groupe phénylsulfonyle, un groupe amino, un groupe diméthylamino, un groupe diéthylamino, un groupe acétylamino ou
un groupe de formule $-OR^8$,
où
$R^8$ représente un atome d'hydrogène, un groupe alkyle en $C_1$-$C_4$, un groupe phényle ou un groupe benzyle

ou

$R^1$ un groupe alkyle en $C_1$-$C_4$,

$R^2$ représente un groupe phényle substitué jusqu'à deux fois, de manière identique ou différente, par un atome de fluor, un atome de chlore, un atome de brome, un groupe cyano, un groupe trifluorométhyle, un groupe trifluorométhoxy, un groupe alcoxy en $C_1$-$C_4$, un groupe méthylthio, un groupe hydroxyle, un groupe méthoxycarbonyle, un groupe éthoxycarbonyle, un groupe diméthylamino, un groupe acétylamino, un groupe diéthylamino,

$R^3$ représente un atome d'hydrogène ou un groupe alkyle en $C_1$-$C_4$,

et

$R^4$ représente un atome d'hydrogène

ainsi que leurs sels,
caractérisé en ce qu'on soumet à une hydrogénation des N-indolyléthyl-sulfonamides de formule générale (II)

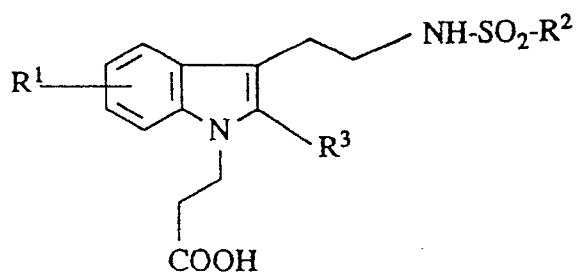

(II)

dans laquelle

$R^1$, $R^2$ et $R^3$ ont la signification mentionnée, ou encore leurs sels, éventuellement en présence d'un solvant organique inerte, en présence d'un acide et d'un agent de réduction, on sépare éventuellement les isomères de la manière habituelle et, dans le cas de la préparation des sels, on fait réagir avec une base correspondante.

5. Procédé pour la préparation des N-dihydroindolyléthyl-sulfonamides de formule générale

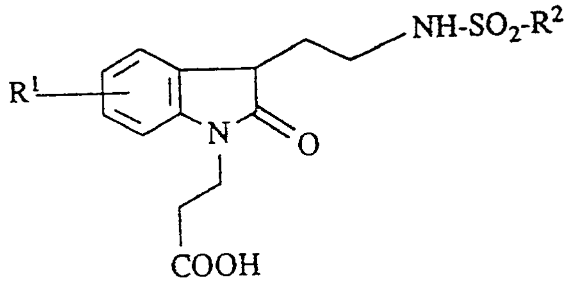

(Ib)

dans laquelle :

$R^1$ représente un atome d'hydrogène, un atome de fluor, un atome de chlore, un atome de brome, un groupe trifluorométhyle, un groupe méthylthio, un groupe éthylthio, un groupe méthylsulfonyle, un groupe éthylsulfonyle, un groupe phénylthio, un groupe phénylsulfonyle, un groupe amino, un groupe diméthylamino, un groupe diéthylamino, un groupe acétylamino ou

un groupe de formule $-OR^8$,

où

$R^8$ représente un atome d'hydrogène, un groupe alkyle en $C_1$-$C_4$, un groupe phényle ou un groupe benzyle

ou

$R^1$ un groupe alkyle en $C_1$-$C_4$,

$R^2$ représente un groupe phényle substitué jusqu'à deux fois, de manière identique ou différente, par un atome de fluor, un atome de chlore, un atome de brome, un groupe cyano, un groupe trifluorométhyle, un groupe trifluorométhoxy, un groupe alcoxy en $C_1$-$C_4$, un groupe méthylthio, un groupe hydroxyle, un groupe méthoxycarbonyle, un groupe éthoxycarbonyle, un groupe diméthylamino, un groupe acétylamino, un groupe diéthylamino,

et de leurs sels

**caractérisé en ce qu'**on oxyde des N-indolyléthylsulfonamides de formule générale

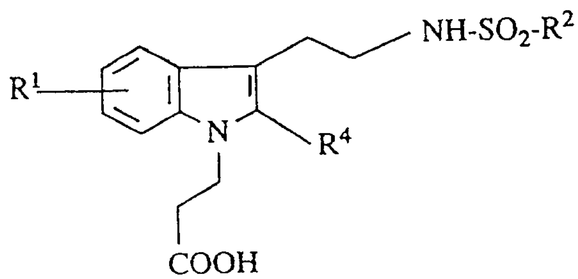

(IIa)

dans laquelle

$R^1$ et $R^2$ ont la signification mentionnée, et

$R^4$ représente un atome d'hydrogène

ou encore leurs sels,

dans un solvant inerte, éventuellement en présence d'un acide et, dans le cas de la préparation des sels, on fait réagir avec une base correspondante.

6. Médicament contenant au moins un N-dihydroindolyléthyl-sulfonamide selon la revendication 1.

7. Médicament selon la revendication 6, contenant de 0,5 à 90 pour cent en poids de N-dihydroindolyléthylsulfonamide, calculé sur le mélange total.

8. Procédé pour la préparation de médicaments selon la revendication 6, **caractérisé en ce qu'**on

amène le N-dihydroindolyléthyl-sulfonamide, éventuellement à l'aide de substances de support et auxiliaires habituelles, à une forme d'application appropriée.

**9.** Utilisation des N-dihydroindolyléthyl-sulfonamides selon la revendication 1 pour la préparation de médicaments.

**10.** Utilisation des N-dihydroindolyléthyl-sulfonamides selon la revendication 1 pour la préparation de médicaments pour le traitement de thromboses, de thrombo-embolies, d'ischémies et de maladies asthmatiques et allergiques.

**Revendications pour l'Etat contractant suivant : ES**

**1.** Procédé pour la préparation de N-dihydroindolyléthyl-sulfonamides de formule générale

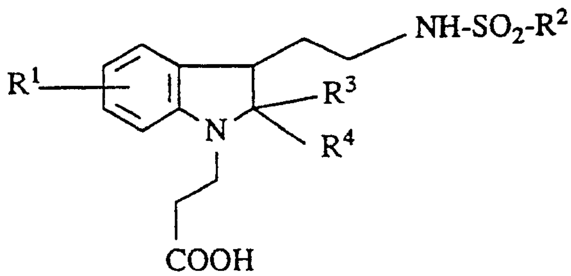

(Ia)

dans laquelle :

$R^1$ représente un atome d'hydrogène, un atome de fluor, un atome de chlore, un atome de brome, un groupe trifluorométhyle, un groupe méthylthio, un groupe éthylthio, un groupe méthylsulfonyle, un groupe éthylsulfonyle, un groupe phénylthio, un groupe phénylsulfonyle, un groupe amino, un groupe diméthylamino, un groupe diéthylamino, un groupe acétylamino ou
un groupe de formule $-OR^8$,
où
$R^8$ représente un atome d'hydrogène, un groupe alkyle en $C_1$-$C_4$, un groupe phényle ou un groupe benzyle

ou

$R^1$ un groupe alkyle en $C_1$-$C_4$,

$R^2$ représente un groupe phényle substitué jusqu'à deux fois, de manière identique ou différente, par un atome de fluor, un atome de chlore, un atome de brome, un groupe cyano, un groupe trifluorométhyle, un groupe trifluorométhoxy, un groupe alcoxy en $C_1$-$C_4$, un groupe méthylthio, un groupe hydroxyle, un groupe méthoxycarbonyle, un groupe éthoxycarbonyle, un groupe diméthylamino, un groupe acétylamino, un groupe diéthylamino,

$R^3$ représente un atome d'hydrogène ou un groupe alkyle en $C_1$-$C_4$,

et

$R^4$ représente un atome d'hydrogène,

et de leurs sels,

**caractérisé en ce qu'**on soumet à une hydrogénation des N-indolyléthyl-sulfonamides de formule générale (II)

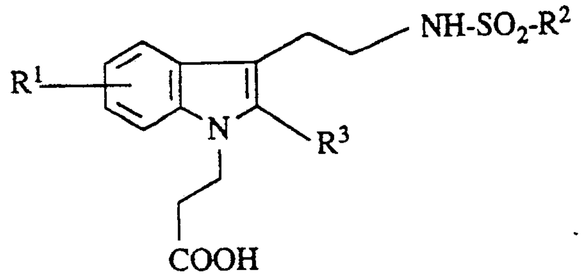

(II)

dans laquelle

$R^1$, $R^2$ et $R^3$ ont la signification mentionnée, ou encore leurs sels, éventuellement en présence d'un solvant organique inerte, en présence d'un acide et d'un agent de réduction, on sépare éventuellement les isomères de la manière habituelle et, dans le cas de la préparation des sels, on fait réagir avec une base correspondante.

2. Procédé selon la revendication 1, **caractérisé en ce qu'**on met en oeuvre, comme acide, des acides carboxyliques ou des acides sulfoniques.

3. Procédé selon la revendication 1, **caractérisé en ce qu'**on met en oeuvre, comme agent de réduction, des hydrures.

4. Procédé selon la revendication 1, **caractérisé en ce qu'**on travaille dans une domaine de température de -40 °C à +80 °C.

5. Procédé selon la revendication 1, **caractérisé en ce qu'**on travaille dans un domaine de température de -20 °C à +60 °C.

6. Procédé pour la préparation de N-dihydroindolyléthyl-sulfonamides de formule générale

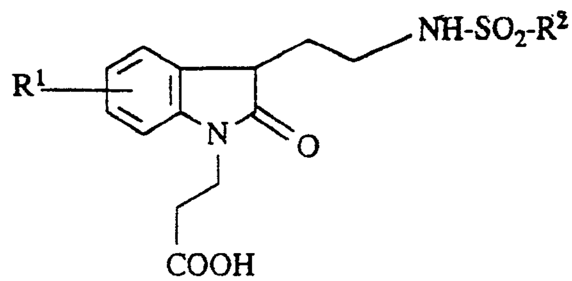

(Ib)

dans laquelle :

$R^1$ représente un atome d'hydrogène, un atome de fluor, un atome de chlore, un atome de brome, un groupe trifluorométhyle, un groupe méthylthio, un groupe éthylthio, un groupe méthylsulfonyle, un groupe éthylsulfonyle, un groupe phénylthio, un groupe phénylsulfonyle, un groupe amino, un groupe diméthylamino, un groupe diéthylamino, un groupe acétylamino ou
un groupe de formule $-OR^8$,
où
$R^8$ représente un atome d'hydrogène, un groupe alkyle en $C_1$-$C_4$, un groupe phényle ou un groupe benzyle

ou

$R^1$ un groupe alkyle en $C_1$-$C_4$,

$R^2$ représente un groupe phényle substitué jusqu'à deux fois, de manière identique ou différente, par un atome de fluor, un atome de chlore, un atome de brome, un groupe cyano, un groupe trifluorométhyle, un groupe trifluorométhoxy, un groupe alcoxy en $C_1$-$C_4$, un groupe méthyl-

thio, un groupe hydroxyle, un groupe méthoxycarbonyle, un groupe éthoxycarbonyle, un groupe diméthylamino, un groupe acétylamino, un groupe diéthylamino,

et de leurs sels

**caractérisé en ce qu'**on oxyde les N-indolyléthylsulfonamides de formule générale

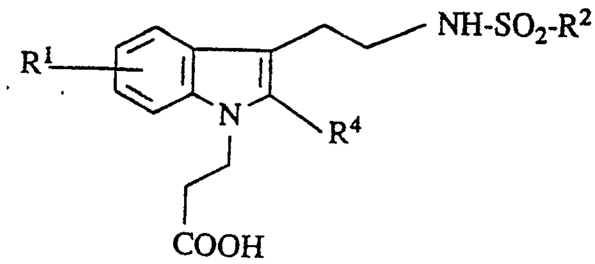

(IIa)

dans laquelle

$R^1$ et $R^2$ ont la signification mentionnée, et

$R^4$ représente un atome d'hydrogène

ou encore leurs sels,

dans un solvant inerte, éventuellement en présence d'un acide et, dans le cas de la préparation des sels, on fait réagir avec une base correspondante.

**7.** Procédé selon la revendication 6, **caractérisé en ce que,** comme agent d'oxydation, on met en oeuvre des dérivés halogénés du N-bromosuccinimide ou encore le diméthylsulfoxyde.

**8.** Procédé selon la revendication 6, **caractérisé en ce que,** comme acide, on met en oeuvre l'acide chlorhydrique, l'acide bromhydrique, l'acide iodhydrique, l'acide sulfurique, l'acide phosphorique, l'acide acétique, l'acide trifluoracétique, l'acide chloracétique, l'acide dichloracétique, l'acide trichloracétique, l'acide méthanesulfonique, l'acide éthanesulfonique, l'acide benzènesulfonique, l'acide toluènesulfonique ou l'acide trifluorométhanesulfonique.

**9.** Procédé selon la revendication 6, **caractérisé en ce qu'**on effectue l'oxydation dans un domaine de température de 0° C à +100° C.

**10.** Procédé selon la revendication 6, **caractérisé en ce qu'**on effectue l'oxydation dans un domaine de température de +20° C à +60° C.